**Europäisches Patentamt**

**European Patent Office**

**Office européen des brevets**

(19)

(11) Veröffentlichungsnummer: **0 346 513**
**A1**

## (12) EUROPÄISCHE PATENTANMELDUNG

(21) Anmeldenummer: **88109558.2**

(22) Anmeldetag: **15.06.88**

(51) Int. Cl.⁴: **A61N 1/32 , A61N 1/04**

(43) Veröffentlichungstag der Anmeldung:
**20.12.89 Patentblatt 89/51**

(84) Benannte Vertragsstaaten:
**AT BE CH DE ES FR GB GR IT LI LU NL SE**

(71) Anmelder: **ETAMA AG**
**Landstrasse 938**
**FL-9496 Balzers(LI)**

(72) Erfinder: **Frick, Kuno**
**Kreuzstrasse 471**
**LI-9496 Balzers(LI)**

(74) Vertreter: **Uri, Peter Alexander, Dipl.-Ing. et al**
**Patentanwälte Strohschänk, Uri & Strasser**
**Innere Wiener Strasse 8**
**D-8000 München 80(DE)**

(54) **Anordnung zur Elektrotherapie.**

(57) Die Erfindung betrifft eine Anordnung zur Elektrotherapie, bei der an einem Träger (4a) mittig ein innerer Pol (3a) angeordnet ist, um den konzentrisch äußeren Pol (2a) liegen, die über ein Schaltgerät (6a) von einer Stromquelle (5a) abwechselnd mit Spannung belegbar sind. Die Stromart und die Schaltabläufe sind beliebig einstellbar. Mit Hilfe der Anordnung können auch nicht exakt zu lokalisierende Stellen ander Hautoberfläche therapiert werden.

## FIG.1

EP 0 346 513 A1

## Anordnung zur Elektrotherapie

Die Erfindung betrifft eine Anordnung zur Elektrotherapie gemäß dem Oberbegriff des Anspruchs 1.

Mit Hilfe von elektrischem Strom wurden bereits vielfältige Versuche unternommen, körperliche Beschwerden, wie Nackenschmerzen, Kopfschmerzen, Trigeminus-Neuralgie, Fazialis Neuralgie, Parkinsonsche Krankheit, Bandscheibenschäden, Bänderrisse, Sportschäden usw., zu behandeln. Die DE-OS 34 37 837 beschreibt beispielsweise ein Gerät zur elektrotherapeutischen Selbstbehandlung des Schmerzes und der Schlaflosigkeit, das mehrere Pole aus unterschiedlichen Metallen und eine Hilfsbatterie aufweist. Solche Geräte können möglicherweise kurzfristig und kurzzeitige Besserungen der Beschwerden bewirken, eine wiederholbare und nachprüfbare Wirkung ist allerdings in der Praxis nicht bewiesen.

In den meisten Fällen ist es nämlich sehr schwierig, mit den Elektroden jene Bereiche an der Hautoberfläche zu erreichen, die geeignet sind, den Strom an diejenigen Stellen im Organismus zu lenken, an denen er seine Heil- bzw. Schmerzlinderungswirkung vollführen soll. Außerdem fließen die durch unterschiedliche Metalle und Batterien erzielbaren Ströme kontinuierlich, wodurch sich keine anregenden Reizeffekte ergeben können, die üblicherweise stets durch zeitlich veränderliche Strome eintreten.

Darüber hinaus ist zu befürchten, daß bestimmte Gewebeteile toxisch belastet werden können, da die Batterie imstande ist, die verschiedenen Metalle im Zusammenwirken mit physiologischen Flüssigkeiten, wie z.B. Schweiß, aufzulösen, und die entstehenden Metallionen in den Organismus transportiert werden können.

Aus der US-PS 4 062 364 ist eine mehrpolige Elektrodenanordnung bekannt, die Wechselstromimpulse aufzubringen imstande ist. Dabei gibt es eine positive und z.B. zwei negative Elektroden, die stets gleichzeitig mit Wechselspannungsimpulsen belegt werden. Diese Elektrodenanordnung ist jedoch auch nicht in ausreichendem Maße geeignet, nicht exakt zu lokalisierende Punkte (z.B. Elektro-Akupunkturpunkte) an der Hautoberfläche zu treffen, so daß häufig die Impulse gewissermaßen nutzlos abgegeben werden und an den Zielgebieten vorbeistreichen. Der Strom wählt bekanntlich denWeg des geringsten Widerstandes, so daß darüber hinaus häufig auch nur eine der negativen Elektroden tatsächlich zu einem Stromfluß führt, während die andere mit einem größeren Widerstand an der Hautoberfläche im wesentlichen stromlos wird. Jedenfalls ist bei dieser Anordnung die Exaktheit der Stromführung nicht gewährleistet.

In Fachkreisen ist des weiteren eine Elektrodenplatte bekannt geworden, die aus einer mehrfach unterteilten Behandlungselektrode und einer Basiselektrode besteht, bei der von einer Stromquelle über ein Schaltgerät zu gleichen oder unterschiedlichen Zeiten im bestimmter oder unbestimmter Reihenfolge Spannungsimpulse auf die Einzelteile der Behandlungselektrode gesandt werden, die sodann die Möglichkeit haben, durch einen dazwischen liegenden Körper auf die Basiselektrode zu treffen. Bei dieser Anordnung wird unter anderem versucht, nach einem "Schrotschußprinzip" im Organismus tieferliegende Gebiete zu erreichen, auch wenn diese nicht exakt lokalisierbar sind. Die Basiselektrode ist dabei zumeist an der der Behandlungselektrode gegenüberliegenden Seite des Körpers anzuordnen.

Dieses bekannte Gerät, bzw. diese bekannte Elektrodenplatte bietet allerdings keine Möglichkeit zur direkten örtlichen Behandlung einzelner, an der Hautoberfläche liegender Punkte bzw. Stellen.

Außerdem ist eine Elektrodenanordnung in Fachkreisen bekannt geworden, die als sogenannte Ohrlektrode bezeichnet wird. Ein getränkter Wattebausch wird im Ohrkanal plaziert; rund um die Ohrmuschel sind ansteuerbare Kontakte vorgesehen, die ebenfalls nach einem "Schrotschußprinzip" Impulse durch das äußere bis zum mittleren und inneren Ohr senden. Bei gleichzeitiger Anordnung von zwei Vorrichtungen (je einer am rechten und am linken Ohr) konnte dieselbe oben erwähnte "Schrotschußwirkung" auch für im Kopfinneren liegende Bereiche erzielt werden.

Aus der US-PS 4 558 704 ist des weiteren eine Elektrodenanordnung bekannt geworden, die einen Ersatz für lahmgelegte Nerven darstellt, indem an bestimmten Stellen eines Körpers Stromimpulse angelegt werden, die die darunterliegenden Muskeln zu Aktivitäten anregen. Eine systematische Behandlung von kleinen begrenzten Gebieten nach dem erwähnten "Schrotschußprinzip" ist jedoch auch mit dieser Anordnung nicht erzielbar.

Der Erfindung liegt die Aufgabe zugrunde, eine Anordnung zur Elektrotherapie zu schaffen, die einfach angewendet werden kann, eine physiologisch wirksame Stromart und Ladungsmenge applizierbar macht und an die Bedürfnisse des jeweiligen Patienten angepaßt werden kann. Insbesondere sollen Gebiete oder Stellen direkt an der Hautoberfläche angeregt (stimuliert) werden können.

Diese Aufgabe wird durch die Merkmale des Anspruchs 1 gelöst.

Die erfindungsgemäße Anordnung ist einfach zu bedienen und die Behandlung (Stimulation) völlig schmerzfrei, wobei insbesondere die individuelle Einstellung der Umschaltfrequenz und/oder der Spannung das Anpassen des Gerätes auf die Bedürfnisse des jeweiligen Patienten ermöglicht. Die mit Hilfe der erfindungsgemäßen Anordnung auf den Organismus aufgebrachten Ströme werden nicht als unangenehme Reizströme empfunden; es wird aber durch sie besonders starke, regulierende und vitalisierende Bio-Energie in den lebenden Organismus "gepumpt". Durch bestimmte, noch nicht restlos geklärte energetische Effekte wird der Körper zur Heilung angeregt und die Zellentätigkeit wird stimuliert, reguliert und normalisiert. Die Stimulation der Zellen erfolgt dabei mit so kleiner Stromstärke, daß das behandelte Gewebe keinerlei Verletzungen, wie z.B. Strommarken, aufweist. Die behandelten Zellen beginnen wesentlich besser zu funktionieren und leiten einen Gesundungsprozeß ein. Dies erfolgt insbesondere auch bei Zellen, deren Lokalität man vor der Behandlung gar nicht genau kennen muß.

Eine Erklärung für die Wirkungsweise der erfindungsgemäßen Anordnung könnte sein, daß die von der Anordnung eingeleiteten Impulse das Einströmen von Nervenreizen über schnell leitende A-Nervenfasern in die Zentren des Mittelhirns bewirken. Da die Schmerzreize hingegen durch langsamleitende C-Nervenfasern in das Mittelhirn transportiert werden, kann der Schmerz unterdrückt werden. Im Thalamus, einer der besonders wichtigen Zwischenstationen auf dem Weg zur Hirnrinde, die der eigentliche Ort der Schmerzempfindung ist, hemmen die schnellen Nervenimpulse die langsamen Schmerzimpulse, die somit die Hirnrinde nicht oder nur abgeschwächt erreichen. Diese sogenannte "Gate control theory of pain" wurde schon vor geraumer Zeit von Melzak und Wal in Kanada erstellt und vielfach ergänzt und bestätigt. Durch die erfindungsgemäße Anordnung ist es nunmehr möglich, die geeigneten Impulse auch in einfacher und funktionssicherer Form zu applizieren.

Neben dieser Theorie fand man auch bereits 1975 körpereigene Stoffe, die (ähnlich dem Morphium) eine stark schmerzlindernde Wirkung besitzen. Man nennt sie "Endorphine". Solche Endorphine werden durch die Wirkung der erfindungsgemäßen Anordnung freigesetzt und bewirken zusätzliche Schmerzlinderung.

In den Unteransprüchen sind bevorzugte Ausführungen der Erfindung näher gekennzeichnet.

Die Ausgestaltung gemäß dem Unteranspruch 2 beruht auf der Erkenntnis, daß es bei verschiedenen Therapieformen vorteilhaft ist, die angegebene Polarität anzuwenden. Durch das Umschalten von einem äußeren Pol zum jeweils benachbarten ergeben sich dabei an den einzelnen, jeweils angesteuerten Polen kurzzeitige Gleichspannungswerte, die auch als Rechteckimpulse bezeichnet werden könnten. Bei der angegebenen Polarität wird vermutet, daß diese der Transportrichtung von Nervenimpulsen und/oder der Freisetzung von Endorphinen förderlich ist.

Die Vorgabe der Fortschaltrichtung bei der Impulsabgabe bzw. der Spannungsanlegung an die einzelnen äußeren Pole erfolgt nach der Art der zu behandelnden Gewebe. Es gibt noch keine eindeutige Erklärung für die Wirkungsweise, wohl aber durch verschiedene Versuche einen Nachweis dafür, daß die Fortschaltrichtung von Bedeutung ist. In Fällen, in denen zu Beginn der Behandlung die Fortschaltrichtung nicht eindeutig festgelegt werden kann, ist die selbsttätige Umkehr der Drehrichtung von größtem Nutzen.

Die Höhe der Umschaltfrequenz ist, wie empirisch festgestellt wurde, ebenso von Bedeutung für die Heilwirkung, wenn es auch dafür noch keine eindeutigen Erklärungen gibt. Vermutlich hängt dies vom allgemein physiologischen Zustand der betreffenden Körperpartien ab, da dieser Zustand das Freisetzen von Endorphinen entweder beschleunigen oder verzögern kann. Ein Anpassen an diese Beschleunigung und Verzögerung kann nun erfindungsgemäß durch die Veränderung der Umschaltfrequenz bewirkt werden.

In vielen Fällen, so wurde festgestellt, ist die geometrische Ungleichheit des inneren und der äußeren Pole vorteilhaft, wobei der Abstand von ca. 2 cm als jener mit dem höchsten Wirkungsgrad bei gleichzeitig bester Treffsicherheit gefunden wurde.

Die erfindungsgemäße Anwendung von Goldelektroden verhindert das Vergiften bestimmter Gewebeteile durch wandernde Metallionen, verbessert die Möglichkeit der klinischen Sauberhaltung der Elektrode und reduziert die Gefahr von Reizungen, wie sie beispielsweise durch Silber entstehen können. Durch die bombierte Ausbildung ist ein optimaler Stromübergang auf die Haut auch an behaarten Körperstellen gewährleistet.

Die integrierten bzw. gedruckten Leiterbahnen erhöhen die Montagegeschwindigkeit für die Elektrode, während die Durchsichtigkeit des Trägers das sichere Positionieren der Elektrode an bestimmten Hautpartien ermöglicht. Ein Arzt kann beispielsweise einen von ihm zuvor mit elektronischen Meßgeräten gefunden Elektroakupunkturpunkt mit einem Farbstift markieren und punktgenau die Elektrode aufsetzen, da er die markierte Stelle durch die Elektrode hindurch betrachten kann.

Bei gleichzeitiger Behandlung von verschiedenen Stellen, z.B. Akupunkturpunkten für linke und rechte Ovarien einer Patientin, ist die Ausbildung des Schaltgerätes für den Anschluß mehrerer Elektroden von Vorteil. Erfahrungsgemäß benötigen bei einem Patienten symmetrisch unterschiedliche Punkte die gleiche Stromform, so daß eine einzige Stromquelle genügt.

Die erfindungsgemäß mögliche willkürliche oder selbsttätige Variation der Stromwerte hilft, die richtige Stromform anzuwenden, wenn eine genaue Analyse vor der Behandlung nicht möglich war. Die Erfahrung zeigt, daß die übrigen nicht treffsicheren Stromformen vermutlich mangels Intensität schadlos verpuffen.

Das Ablesen der Werte an dem Meßgerät, bzw. an dem Oszilloskop ist einerseits für den behandelnden Arzt, bzw. für dessen Therapieprotokoll von Bedeutung, andererseits erkennt der Patient jedoch auch selbst, daß hier bestimmte Impulse an seinen Körper angelegt werden, was ihn selbst geistig zur Heilung stimuliert.

Die im Rahmen der Erfindung optimalen Frequenzwerte liegen im Bereich von 0,5 bis 20 Hertz; sie führen am einfachsten durch eine automatische Veränderung der Umschaltfrequenz zu großer Treffsicherheit und zum Therapieerfolg, wobei die im physiologischen Bereich liegenden Ströme in einem Wertebereich von etwa 1 bis 1000 Mikroampere zwar nur schwer meßbar, jedoch therapeutisch sehr wirksam sind.

Bei der am menschlichen Körper üblicherweise vorliegenden Beschaffenheit der Hautoberfläche und ihrer Leitfähigkeit ist es zur Erzielung der gewünschten Ströme bevorzugt, daß die Stromquelle im Spannungsbereich von etwa 1,2 bis 7 V einstellbar ist. Bevorzugt sind Spannungswerte von 1,7 bis 1,8 V.

Die einfache Transportierbarkeit und kompakte Bauweise insbesondere bei vollständiger Transistorisierung ist durch die Ausführung des letzten Unteranspruchs gewährleistet.

Die Erfindung wird im folgenden beispielsweise unter Bezugnahme auf die Zeichnung näher erläutert; es zeigt:

Fig. 1 eine erste Ausführungsform der Anordnung mit einer Elektrode mit drei kreisringsektorförmigen Außenpolen;

Fig. 2 eine zweite Ausführungsform der Anordnung mit einer Elektrode mit vier kreisförmigen äußeren Polen;

Fig. 3 eine dritte Ausführungsform der Anordnung mit einer Elektrode, bei der zwölf äußere kreisförmige Pole den Innenpol auf einem zum Mittelpunkt des Innenpols konzentrischen Kreis umgeben; und

Fig. 4 ein schematisches Blockdiagramm der Einheit aus Stromquelle und Schaltgerät.

In der Zeichnung sind gleiche Teile mit gleichen Bezugszeichen, ähnliche Teile mit gleichen Bezugszeichen aber unterschiedlichen Buchstaben-Indices bezeichnet. Die Figuren werden übergreifend beschrieben.

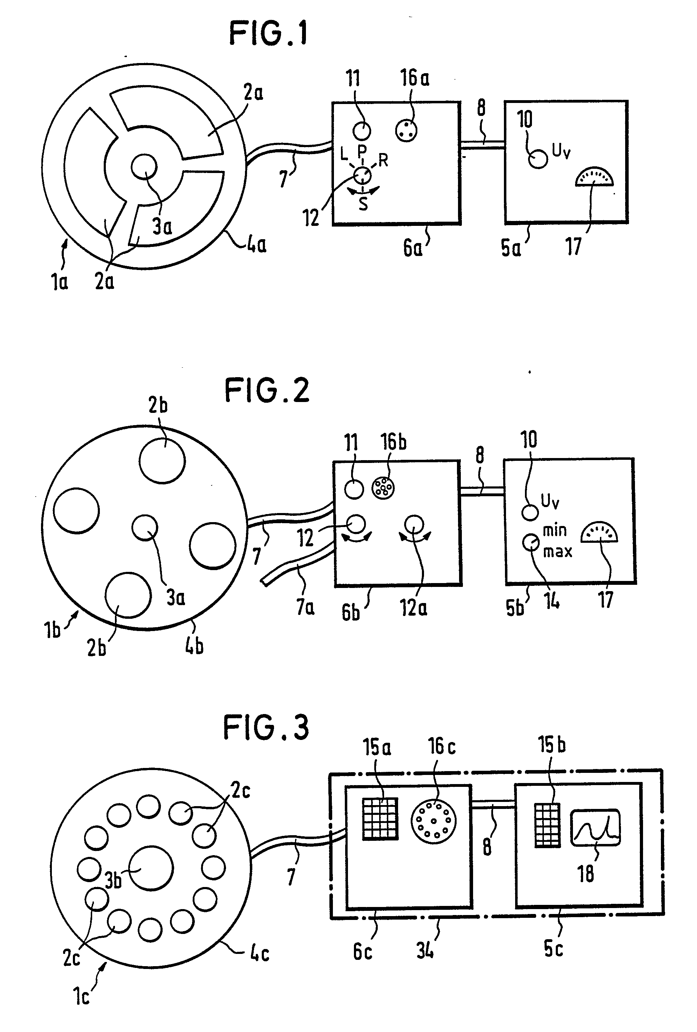

Wie in den Fig. 1 bis 3 gezeigt ist, sind die Elektroden 1 für verschiedene Anwendungen entsprechend unterschiedlich ausgebildet. Die Elektrode 1a der Fig. 1 verfügt über nur drei äußere Pole 2a, die als Kreisringsektoren ausgebildet und konzentrisch um den inneren Pol 3a herum angeordnet sind. Sie decken großflächig den zu behandelnden Bereich des Körpers ab, wobei der tatsächliche Stromübertritt in Abhängigkeit von der lokal variablen Leitfähigkeit der Hautoberfläche jeweils nur an einzelnen Bereichen der Pole 2a, 3a stattfindet.

Unterschiedlich davon sind die beispielhaften Anordnungen mit vier äußeren Polen 2b gemäß der Fig. 2, bzw. mit zwölf äußeren Polen 2c gemäß der Fig. 3.

Sämtliche Pole 2 und 3 sind jeweils auf einem Träger 4a bis 4c montiert, der zur besseren Lokalisierung des zu behandelnden Körperbereichs durchsichtig ist. Der Träger ist vorzugsweise aus Acrylglas hergestellt, die Pole bestehen aus Metallblech, beispielsweise Goldblech oder einem Blech aus Goldlegierung und sind auf einer Oberfläche des Trägers 4 befestigt. Die elektrische Zuleitung zu den einzelnen Polen erfolgt über Leitbahnen nach Art einer gedruckten Schaltung (in der Zeichnung nicht dargestellt). Der Anschluß der Elektroden an das Schaltgerät 6 und die Stromquelle 5 erfolgt jeweils über ein mehradriges Anschlußkabel 7.

Das Schaltgerät 6a gemäß der Fig. 1 verfügt über einen Drehknopf 11 zur Einstellung der Umschaltfrequenz, ferner über eine LED-Anzeige 16a, die zu den Polen 2a der Elektrode 1a parallel geschaltet ist und den jeweiligen Ansteuerungszustand der Pole 2a angibt. Das bedeutet, daß eine LED (Leuchtdiode) der Anzeige 16a immer dann aufleuchtet, wenn der mit ihr verbundene äußere Pol der Elektrode 1a unter Spannung gesetzt ist. Ferner ist ein Drehknopf 12 zur Wahl der gewünschten Fortschaltrichtung vorgesehen. In der Stellung L erfolgt die Fortschaltrichtung entgegen dem Uhrzeigersinn, in der Stellung R im Uhrzeigersinn und in der Stellung P erfolgt gar kein Fortschalten, es werden vielmehr alle äußeren Pole 2a gleichzeitig unter Spannung gesetzt. Bei diesem letztgenannten Schaltzustand sucht sich der Strom in Abhängigkeit von der lokal unterschiedlichen Leitfähigkeit der Haut in dem betreffenden Gebiet seinen Weg selbst. Darüber hinaus besitzt der Dreh knopf 12 eine Stellung S, in welcher eine selbsttätige Umkehr der Fortschaltrichtung nach Durchlauf der einzelnen Pole erfolgt.

In der Fig. 2 ist das Schaltgerät 6b mit einem doppelten Kabel 7, 7a ausgeführt, damit gleichzeitig zwei einander gleiche Elektroden 1b angesteuert werden können. Bei dieser Ausführung gemäß Fig. 2 umfassen die Elektroden 1b jeweils vier äußere Pole 2b, die auf einem zum Mittelpunkt des inneren Pols 3a konzentrischen Kreis gleichmäßig verteilt sind.

Das Schaltgerät 6c gemäß der Fig. 3 verfügt über einen programmierbaren Computer mit einem Programmeingabefeld 15a zur willkürlichen Bestimmung der jeweiligen Schaltfolge, in der die äußeren Pole 2c der Elektrode 1c mit Strom angesteuert werden.

Das Schaltgerät 6 ist bei allen Ausführungsbeispielen jeweils über eine Leitung 8 mit der Stromquelle 5 verbunden. Wie aus der Fig. 3 ersichtlich ist, sind vorzugsweise das Schaltgerät 6c und die Stromquelle 5c in einem eigenen Gehäuse 34 integriert.

Die Stromquelle 5a der ersten Ausführungsform gemäß Fig. 1 besitzt in ihrer einfachsten Ausbildung einen Drehknopf 10 zur Einstellung der an die Pole geführten Spannung Uv, sowie über ein Meßgerät 17 zum Anzeigen der jeweils eingestellten Spannungswerte.

Die Stromquelle 5b gemäß der Fig. 2 verfügt darüber hinaus über einen Drehknopf 14 für die Minimum- und Maximum-Einstellung der Spannung Uv.

Die Stromquelle 5c gemäß der Fig. 3 ist vollständig und in allen betreffenden Parametern über ein Programmeingabefeld 15b programmierbar. Ein Oszilloskop 18 zeigt die aufgrund der Programmierung abgegebenen elektrischen Parameter an.

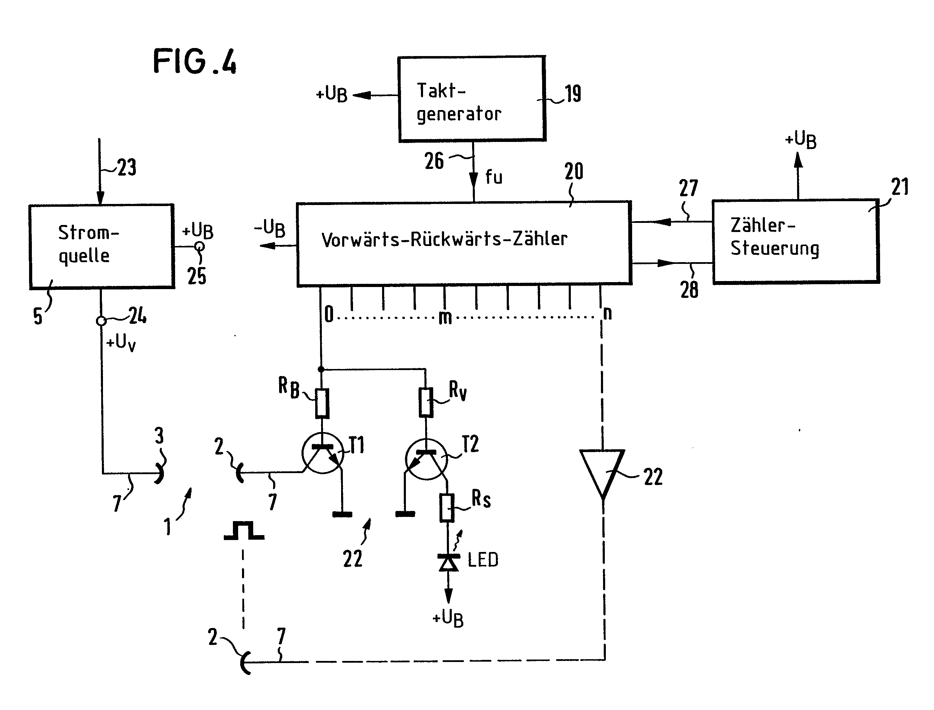

In der Fig. 4 ist als Blockschaltbild der Zusammenbau einer Elektrode 1, einer Stromquelle 5 und des Steuergeräts 6 dargestellt.

Das Steuergerät 6 umfaßt einen Taktgenerator 19, einen Zähler 20, eine Zählersteuerung 21 und eine Vielzahl von Treiberanordnungen 22 für jeden Ausgang des Zählers 20. Die Ausgänge der Treiberschaltungen 22 sind über die Adern des Kabels 7 jeweils mit einem der äußeren Pole 2 der Elektrode 1 verbunden.

Die Stromquelle 5 ist in herkömmlicher, dem Fachmann vertrauter Weise aufgebaut. Sie enthält eine Spannungsquelle für die konstante Betriebsspannung $U_B$ der elektronischen Bauelemente des Schaltgeräts 6. Diese konstante Betriebsspannung $U_B$ wird am Ausgang 25 der Stromquelle abgegeben. Ferner umfaßt die Stromquelle 5 eine zweite Spannungsquelle, deren Ausgangsspannung Uv einstellbar variabel ist und am Ausgang 24 der Stromquelle 5 abgegeben wird. Der Einstellungsbereich der variablen Spannung $U_v$ beträgt etwa 1,2 V bis 7,0 V. Vorzugsweise ist der Wert Uv auf 1,7 V bis 1,8 V eingestellt.

Wie dargestellt, ist der Ausgang 24 der Stromquelle 5 über eine Ader des Kabels 7 zur Zufuhr der positiven Polarität von Uv unmittelbar mit dem inneren Pol 3 der Elektrode 1 ver bunden. Der Ausgang 25 ist jeweils mit den Stromversorgungsanschlüssen der einzelnen Komponenten des Schaltgeräts 6 zur Zufuhr der positiven Betriebsspannung $U_B$ verbunden. Im Blockschaltbild ist dies nur angedeutet, die einzelnen Zufuhrleitungen sind dabei weggelassen, um die Zeichnung übersichtlicher zu halten. Die Energieversorgung der Stromquelle 5 erfolgt über den Eingang 23 aus dem Stromnetz oder alternativ aus einer Batterie.

Der Taktgenerator 19 des Schaltgeräts 6 ist ebenfalls in herkömmlicher und bekannter Weise aufgebaut. Der Taktgenerator 19 gibt an seinem Ausgang 26 Rechteckimpulse mit einer Wiederholfrequenz fu ab, die dem Zähleingang des Zählers 20 zugeführt sind. Die vom Taktgenerator 19 abgegebene Frequenz kann im Bereich von etwa 0,5 Hertz bis 20 Hertz eingestellt werden.

Der Zähler 20 ist als Vorwärts-Rückwärts-Zähler mit n Ausgängen aufgebaut. Die Anzahl n der Ausgänge ist mindestens so groß wie die Anzahl der anzusteuernden äußeren Pole 3 der verwendeten Elektrode 1.

Bei Ansteuerung durch den Taktgenerator 19 zählt der Zähler 20 die zugeführte Anzahl von Taktimpulsen derart, daß jeweils an einem seiner Ausgänge 0 bis n ein Signal erscheint. Die Zählrichtung des Zählers 20 kann über die Leitung 27 derart beeinflußt werden, daß der Zähler 20 entweder vom Ausgang 0 über den Ausgang m in steigender Folge bis zum letzten Ausgang n zählt, oder aber in umgekehrter Reihenfolge. Es ist auch möglich, über die Leitung 27 den Zähler 20 derart zu steuern, daß er zunächst vom Ausgang 0 bis zu einem wählbaren Ausgang m zählt, dann seine Zählrichtung umkehrt und wieder bis zum Ausgang 0 zurückzählt, hier wieder seine Zählrichtung umkehrt, usw. Zur Steuerung der gewünschten Zählweise ist die Zählersteuerung 21 vorgesehen, die über die Leitung 28 den gerade erreichten Zählerstand des Zählers, d.h. den gerade mit einem Signal beaufschlagten Ausgang m feststellt und dann je nach ihrer Einstellung über die Leitung 27 den Befehl abgibt, entweder bis zu einem weiteren vorbestimmbaren Ausgang weiterzuzählen, beispielsweise bis zum letzten Ausgang n und dann rückwärts zu zählen, oder aber dies bereits bei einem mittleren Ausgang m zu tun. Es ist auch möglich, die Zählersteuerung derart einzustellen, daß stets eine Zählfolge von 0 bis n eingehalten wird, die stets wieder bei 0 beginnt. Der Aufbau einer derartigen Zählersteuerung ist kein zentraler Bestandteil der Erfindung und im übrigen für den Elektronikfachmann bekannt.

Jeder der einzelnen Ausgänge 0 bis n des Zählers 20 steuert eine Treiberanordnung 22 an. In der Zeichnung ist die Treiberanordnung nur für den Ausgang 0 im einzelnen dargestellt und symbolisch zusätzlich für den letzten Ausgang n. Die den zwischenliegenden Ausgängen des Zählers 20 zugeordneten, völlig gleich aufgebauten Treiberanordnungen 22 sind der Übersichtlichkeit halber weggelassen.

Wie im einzelnen für den Ausgang 0 dargestellt ist, umfaßt jede Treiberanordnung 22 zwei Treiberschaltungen. Die eine Treiberschaltung umfaßt einen über einen Basiswiderstand $R_B$ angesteuerten Transistor T1, dessen Emitter mit Masse und dessen Kollektor über eine Ader des Kabels 7 mit einem äußeren Pol 2 einer Elektrode 1 verbunden ist. Wenn der Zähler 20 den Zählwert 0 erreicht hat, und daher an seinem Ausgang 0 ein Signal anliegt, wird der Transistor T1 durchgeschaltet, so daß über das Kabel 7 der angeschlossene äußere Pol 2 an Masse gelegt wird und daher zwischen dem inneren Pol 3 und dem äußeren Pol 2 etwa die Spannung + Uv herrscht. Parallel zu diesem ersten Treiber ist an den Ausgang 0 innerhalb der Treiberanordnung 22 ein zweiter Treiber vorgesehen, der aus dem Vorwiderstand Rv und dem Transistor T2 besteht. Dieser Transistor T2 wird über den Vorwiderstand Rv an seiner Basis angesteuert. Sein Emitter ist mit Masse verbunden, während sein Kollektor über einen Schutzwiderstand an eine Leuchtdiode LED der LED-Anzeige 16 geführt ist. Die Leuchtdiode LED ist mit einer Elektrode mit der festen Betriebsspannung + $U_B$ verbunden. Wenn also der Zähler 20 den Zählwert 0 erreicht und daher an seinem Ausgang 0 ein Signal anliegt, wird auch der Transistor T2 durchgeschaltet, die mit ihm verbundene Leuchtdiode LED wird mit Strom versorgt und leuchtet auf. Auf diese Weise wird auf der LED-Anzeige angezeigt, welcher äußere Pol der Elektrode 1 gerade angesteuert ist, und wie die Fortschaltung der Spannung an den äußeren Polen erfolgt.

**Ansprüche**

1. Anordnung zur Elektrotherapie mit einer Stromquelle (5), einem damit verbundenen Schaltgerät (6) und einer über ein Kabel (7) anschließbaren mehrpoligen Elektrode (1) auf einem Träger (4), dadurch **gekennzeichnet**, daß mindestens drei äußere Pole (2) der Elektrode (1) auf einem zum Mittelpunkt des inneren Pols (3) konzentrischen Kreis angeordnet sind, daß die äußeren Pole (2) von dem Schaltgerät (6) alternierend mit einer bestimmten Umschaltfrequenz mit einer Polarität unter Spannung setzbar sind, während der innere Pol (3) die andere Polarität trägt, und daß die Spannung Uv der Stromquelle (5) und die Umschaltfrequenz fu des Schaltgeräts (6) veränderbar sind.

2. Anordnung nach Anspruch 1, dadurch **gekennzeichnet**, daß die äußeren Pole (2) gegenüber dem inneren Pol (3) mit negativer Polarität unter Spannung gesetzt sind.

3. Anordnung nach Anspruch 1 oder 2, dadurch **gekennzeichnet**, daß die äußeren Pole (2) nacheinander wahlweise im oder entgegen dem Uhrzeigersinn unter Spannung setzbar sind, und/oder daß sich die Spannungsansteuerungsfolge selbsttätig bei Erreichen vorgegebener Pole (2) umkehrt.

4. Anordnung nach einem der Ansprüche 1 bis 3, dadurch **gekennzeichnet**, daß die Umschaltfrequenz fu einstellbar und/oder nach einem vorgegebenen Programm selbsttätig veränderbar ist, und/oder daß die Schaltvorgänge an einem LED-Display sichtbar gemacht sind.

5. Anordnung nach einem der vorhergehenden Ansprüche, dadurch **gekennzeichnet**, daß die äußeren Pole (2) und der innere Pol (3) unterschiedliche Flächenmaße aufweisen, wobei vorzugsweise der Abstand zwischen dem inneren Pol (3) und den äußeren Polen (2) etwa 2 cm beträgt.

6. Anordnung nach einem der vorhergehenden Ansprüche, dadurch **gekennzeichnet**, daß die Pole (2, 3) im wesentlichen aus Gold oder einer Goldlegierung bestehen und/oder von dem Träger (4) weg konvex bombiert sind.

7. Anordnung nach einem der vorhergehenden Ansprüche, dadurch **gekennzeichnet**, daß die Pole (2, 3) durch im Träger (4) integrierte, vorzugsweise gedruckte Leiterbahnen mit dem Kabel (7) verbunden sind, und/oder daß der Träger (4) vorzugsweise aus transparentem Kunststoff, z.B. aus Acrylglas aufgebaut ist.

8. Anordnung nach einem der vorhergehenden Ansprüche, dadurch **gekennzeichnet**, daß das Schaltgerät (6) für den Anschluß von mehr als einer Elektrode (1) ausgebildet ist.

9. Anordnung nach einem der vorhergehenden Ansprüche, dadurch **gekennzeichnet**, daß die Veränderung der Spannung der Stromquelle (5) und/oder der Umschaltfrequenz des Schaltgeräts (6) nach einem Programm selbsttätig änderbar ist, und/oder daß zumindest die minimalen und maximalen Spannungswerte der Stromquelle (5) einstellbar sind, wobei die jeweiligen Werte an einem, vorzugsweise eingebauten Meßgerät (17) oder an einem Oszilloskop (18) ablesbar sind.

10. Anordnung nach einem der vorhergehenden Ansprüche, dadurch **gekennzeichnet**, daß die Umschaltfrequenz fu des Schaltgeräts (6) zwischen 0,5 und 20 Hertz veränderbar ist, und daß die von der Stromquelle (5) abgegebene Stromstärke im Bereich von 1 bis 1000 µA liegt.

11. Anordnung nach einem der vorhergehenden Ansprüche, dadurch **gekennzeichnet**, daß die Spannung Uv der Stromquelle (5) im Bereich von etwa 1,2 V bis 7,0 V einstellbar ist und vorzugsweise 1,7 V bis 1,8 V beträgt.

12. Anordnung nach einem der vorhergehenden Ansprüche, dadurch **gekennzeichnet**, daß das Schaltgerät (6) und die Stromquelle (5) gemeinsam in einem Gehäuse (34) untergebracht sind.

FIG.1

FIG.2

FIG.3

FIG.4

Zähler-Steuerung 21

+U_B

27 28

22

Takt-generator 19

26 fu

Vorwärts-Rückwärts-Zähler 20

−U_B

n

m

0

R_v  T2  R_s  LED  +U_B

R_B  T1  22

2  7

2

7

2

Strom-quelle 5

+U_B 25

23

24 +U_v

3

1

7

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 437 346 (THOMA)<br>* Seite 5, Zeilen 1-30; Figur *<br>--- | 1,3,4,<br>10 | A 61 N 1/32<br>A 61 N 1/04 |
| A | EP-A-0 145 176 (WACO CORP. OVERSEAS LTD.)<br>* Seite 9, Zeilen 2-11; Figur 10 *<br>--- | 1,2,5-7 | |
| A | EP-A-0 029 245 (SIEMENS)<br>* Seite 6, Zeilen 17-28; Figur 1 *<br>--- | 1 | |
| A | EP-A-0 268 701 (JAMUTRON)<br>----- | | |

RECHERCHIERTE SACHGEBIETE (Int. Cl.4)

A 61 N
A 61 B

Der vorliegende Recherchenbericht wurde für alle Patentansprüche erstellt

| Recherchenort | Abschlußdatum der Recherche | Prüfer |
|---|---|---|
| DEN HAAG | 30-01-1989 | SCHMIERER U.J. |